(19) Europäisches Patentamt
European Patent Office
Office européen des brevets

(11) Veröffentlichungsnummer: **0 094 633**
**B1**

(12) **EUROPÄISCHE PATENTSCHRIFT**

(45) Veröffentlichungstag der Patentschrift: **09.11.88**

(51) Int. Cl.⁴: **C 07 D 209/54** // C07C93/12, C07C121/46

(21) Anmeldenummer: **83104713.9**

(22) Anmeldetag: **13.05.83**

(54) **Spiro-2-aza-alkan-3-carbonitrile, Verfahren zu ihrer Herstellung und ihre Verwendung.**

(30) Priorität: **17.05.82 DE 3218540**

(43) Veröffentlichungstag der Anmeldung:
**23.11.83 Patentblatt 83/47**

(45) Bekanntmachung des Hinweises auf die Patenterteilung:
**09.11.88 Patentblatt 88/45**

(84) Benannte Vertragsstaaten:
**AT BE CH DE FR GB IT LI NL SE**

(56) Entgegenhaltungen:
**EP-A-0 050 800**
**DE-A-2 756 360**

(73) Patentinhaber: **HOECHST AKTIENGESELLSCHAFT**
**Postfach 80 03 20**
**D-6230 Frankfurt am Main 80 (DE)**

(72) Erfinder: **Teetz, Volker, Dr.**
**An der Tann 20**
**D-6238 Hofheim am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 094 633 B1

Courier Press, Leamington Spa, England.

**Beschreibung**

Die Erfindung betrifft Verbindungen der Formel I

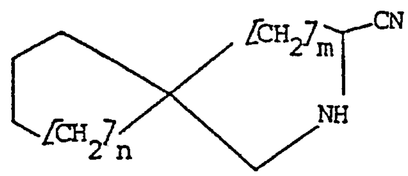

(I)

in welcher m eine ganze Zahl von 1 bis 3 und n eine ganze Zahl von 1 bis 4 bedeuten. Besonders bevorzugt sind Verbindungen der Formel I, in der m=1 ist.

Die Erfindung betrifft weiterhin ein Verfahren zur Herstellung von Verbindungen der Formel I, das dadurch gekennzeichnet ist, daß man Verbindungen der Formel II

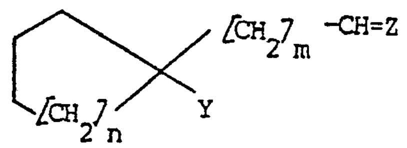

(II)

in welcher m und n die vorstehend genannten Bedeutungen haben, Y für Aminomethyl und Z für eine sauer abspaltbare, gegen metallorganische Reagentien inerte oxo-Schutzgruppe stehen, aufeinanderfolgend mit a) Säuren und b) Cyanwasserstoff oder Cyaniden behandelt.

Bevorzugte Schutzgruppen Z der Oxofunktion sind Gruppen der Formel $(OR^1)_2$, wobei $R^1$ Alkyl mit 1 bis 6 vorzugsweise 1 bis 4 C-Atomen bedeutet oder die beiden Reste $R^1$ gemeinsam Alkylen mit 2 bis 5 C-Atomen bedeuten. Für die Umsetzung der bevorzugten Acetale der Formel II ($Y=CH_2NH_2$) haben sich verdünnte Mineralsäuren wie Salzsäure oder Schwefelsäure aber auch organische Säuren, wie Essigsäure, Oxal- oder Weinsäure als geeignet erwiesen. Die Behandlung der geschützten Verbindungen der Formel II ($Y=CH_2NH_2$) mit starken Säuren, bevorzugt Salzsäure führt unter Abspaltung der Schutzgruppe zum Aldimin, an das anschließend unter Bedingungen analog der Strecker-Synthese, Cyanwasserstoff angelagert wird.

Es wurde gefunden, daß die Umsetzung mit Cyanwasserstoff in essigsaurer Lösung wesentlich besser und in hoher Ausbeute verläuft.

Neben Cyanwasserstoff sind auch Cyanide, vorzugweise Alkali- und Erdalkalicyanide geeignet.

Man erhält die vorstehend genannten Verbindungen der Formel II, in der m, n, Y und Z die vorstehend genannten Bedeutungen haben ausgehend von Cycloalkancarbonitrilen der Formel IV,

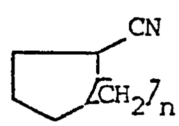

(IV)

in der n die vorstehend genannte Bedeutung hat, indem man diese in α-Stellung metalliert und mit einem geschützten ω-Halogenaldehyd der Formel $X—[CH_2]_m—CH=Z$, in der m und Z vorstehende Bedeutung haben und X für Brom oder Chlor steht, wobei Acetale bevorzugt sind, unter Bildung einer Verbindung der Formel II, in der m, n und Z vorstehende Bedeutung haben und Y für Cyan steht, umsetzt. Die anschließende Reduktion führt zu den Aminomethylverbindungen der Formel II. Das hier beschriebene Verfahren geht von einfacheren und gut zugänglichen Vorprodukten aus und liefert auf jeder Stufe Ausbeuten von über 80%.

Die α-Metallierung der Nitrile der Formel IV kann mit bekannten Reagentien vorgenommen werden, geeignete sind z.B. Kaliumamid in flüssigem Ammoniak, Lithiumdiethylamid in HMPT, oder bevorzugt in Hexan. Eine entsprechende Lösung kann nach bekannten Verfahren (siehe beispielsweise Houben-Weyl, Methoden der Organischen Chemie) hergestellt werden. Als Alkylierungsreagentien kommen beliebige aliphatische Chlor- und Brom-Verbindungen von geschützten Aldehyden vorzugsweise Acetale in Frage. Die Wahl des Acetals ist für die Reaktion nicht kritisch, so daß z.B. Dimethyl- und Diethylacetale aber auch cyclische wie Ethylenacetale und höhere Homologe einsetzbar sind. Die Reduktion der Nitrilgruppe erfolgt am besten mit metallischem Natrium in Alkohol. Eine katalytische Reaktion mit Raney-Nickel ist ebenfalls möglich, jedoch sollte in Essigsäureanhydrid in Gegenwart von Basen gearbeitet werden. Die entstehenden N-Acetyl-Derivate der Verbindungen der Formel II können ebenfalls in den Nachfolgereaktionen eingesetzt werden.

Aus Beispiel 36 der EP—A—50 800 ist 2-(1-Cyanocyclopentyl)-acetaldehyd-diethyl-acetal und 2-(1-aminomethylcyclopentyl)-acetaldehyd-diethyl-acetal bekannt. Weiterhin werden in Tetrahedron Letters *1975*, 3852 die Verbindungen 2-(1-Cyanocyclohexyl)-acetaldehyd-1,3-dioxetan und 3-(1-Cyanocyclohexyl)-propionaldehyd-diethyl-acetal beschrieben.

Der Erfindung betrifft auch Verbindungen der Formel II, in welcher Z, m und n die oben genannten Bedeutungen haben und Y Aminomethyl oder Cyan bedeutet, wobei die aus EP—A—50800 und Tetrahedron Letters *1975*, 3852 bekannten, vorstehend erwähnten Verbindungen ausgenommen sind. Bevorzugt sind Verbindungen der Formel II, in der m=1 bedeutet. Besonders bevorzugt sind auch solche Verbindungen der Formel II, in der Y für Aminomethyl steht.

Die Erfindung betrifft ferner die Verwendung von Verbindung der Formel I, in welcher m und n die vorstehend genannten Bedeutungen haben, in einem Verfahren zur Herstellung von Verbindungen der Formel III

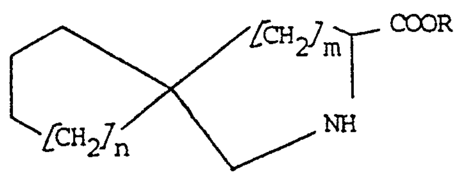

(III)

in welcher m und n die vorstehend genannten Bedeutungen haben und R für Wasserstoff, Alkyl mit 1 bis 10 C-Atomen, Aralkyl mit 7 bis 9 C-Atomen, Cycloalkyl mit 5 bis 10 C-Atomen, Alkylcycloalkyl mit 6 bis 12 C-Atomen oder Cycloalkylalkyl mit 6 bis 12 C-Atomen steht durch Solvolyse mit einer Verbindung der Formel ROH, in welcher R vorstehende Bedeutung hat.

Die Verseifung des Nitrils der Formel I kann nach an sich bekannten Methoden erfolgen. Als besonders geeignet hat sich die Verseifung mit Mineralsäuren wie beispielsweise 4 n Salzsäure erwiesen. Aus den entstandenen Aminosäuren können nach den üblichen Methoden der Peptidchemie die Ester hergestellt werden. Als besonders günstig erwies es sich jedoch, die Aminonitrile unter Säurekatalyse (z.B. HCl-Gas) in wasserfreiem Medium mit entsprechenden Alkoholen direkt zu den Estern umzusetzen. Dieses Verfahren wird bevorzugt.

Bevorzugt ist die Verwendung von Verbindungen der Formel I zur Herstellung von Verbindungen der Formel III, in der R Wasserstoff, Alkyl mit 1 bis 10 C-Atomen, Aralkyl mit 7 bis 9 C-Atomen insbesondere Benzyl und Nitrobenzyl und Alkylcycloalkyl mit 6 bis 10 C-Atomen bedeuten, wobei die Reste R auch chiral sein können. Als Beispiel für einen chiralen Rest Rest R sei Menthyl genannt.

Die Verbindungen der Formeln I und III sind Zwischenprodukte zur Herstellung von Verbindungen der Formel V,

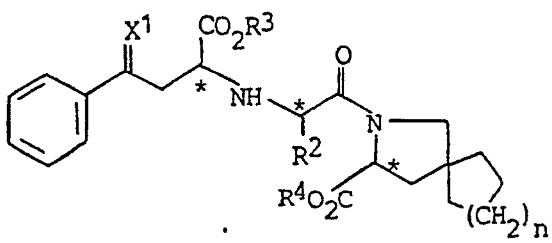

(V)

in welcher

n 1, 2, 3 oder 4,

$R^2$ einen Alkylrest einer natürlich vorkommenden Aminosäure HOOC—CH($NH_2$)—$R^1$,

$R^3$ Wasserstoff, Alkyl mit 1 bis 6 C-Atomen oder gegebenenfalls nitrosubstituierter Aralkyl mit 7 bis 9 C-Atomen,

$R^4$ Wasserstoff, Alkyl oder Cycloalkyl mit 1 bis 10 C-Atomen oder gegebenenfalls nitrosubstituiertes Aralkyl mit 7 bis 9 C-Atomen und

$X^1$ 2 Wasserstoffatome oder 1 Sauerstoffatom bedeuten, sowie deren physiologisch verträglichen Salze mit Säuren und, falls $R^2$ und/oder $R^3$ Wasserstoff bedeuten, mit Basen.

In Formel V können das C-Atom in Position 3 der Spirocyclus und die mit einem Stern (*) markierten C-Atome der Kette sowohl R- als auch S-Konfiguration aufweisen. Bevorzugt sind jedoch Verbindungen, deren C-Atom in Position 3 des Spirocyclus und deren mit einem Stern markierten C-Atome der Kette jeweils die S-Konfiguration aufweisen.

Das Verfahren zur Herstellung von Verbindungen der Formel V ist dadurch gekennzeichnet, daß man Verbindungen der Formel VI

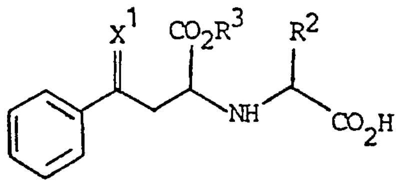

(VI)

in welcher $R^2$, $R^3$ und $X^1$ die vorstehend angegebenen Bedeutungen haben mit Verbindungen der Formel III, in welcher R und n die vorstehend angegebenen Bedeutungen mit Ausnahme der von R=Wasserstoff haben und m für 1 steht, kondensiert und anschließend gegebenenfalls die Reste $R^3$ und/oder $R^4$ hydrogenolytisch, sauer oder basisch abspaltet und die erhaltenen Verbindungen der Formel V gegebenenfalls in ihre physiologisch verträglichen Salze überführt.

Die Kondensation der Verbindungen der Formel VI mit den Estern der Formel III erfolgt vorzugsweise nach bekannten Verfahren der Peptidchemie. Besonders bevorzugt sind solche Verfahren, die ausreichend Schutz vor Racemisierung bieten, wie z.B. die DCC/HOBt-Methode oder die in der DE—OS 29 01 843 beschriebenen Alkanphosphonsäureanhydrid-Methode.

Verbindungen der Formel V, in denen wenigstens einer der Reste $R^3$ und $R^4$ Wasserstoff ist, können nach bekannten Methoden in die Ester der Formel V überführt werden, in denen $R^3$ und $R^4$ die oben genannte Bedeutung mit Ausnahme der von Wasserstoff haben.

Die Verbindungen der allgemeinen Formel V besitzen eine langandauernde, intensive blutdrucksenkende Wirkung. Sie werden nach peroraler Gabe gut resorbiert und können zur Bekämpfung des Bluthochdrucks verschiedener Genese eingesetzt und für sich oder in Kombination mit anderen blutdrucksenkenden, gefäßerweiternden oder diuretisch wirksamen Verbindungen angewandt werden. Die Anwendung kann intravenös, subcutan oder peroral erfolgen, die perorale Gabe wird bevorzugt. Die Dosierung bei peroraler Gabe liegt bei 0,1—100 mg je Einzeldosis.

Sie kann in schweren Fällen auch erhöht werden, da toxische Eigenschaften bisher nicht beobachtet wurden. Auch eine Herabsetzung der Dosis ist möglich und vor allem dann angebracht, wenn gleichzeitig Diuretika verabreicht werden. Bei intravenöser oder subcutaner Verabreichung sollte die Einzeldosis zwischen 0,01 und 20 mg liegen.

Im Tierversuch wurden z.B. folgende Wirksamkeiten erhalten: Es wurde die Unterdrückung des durch 310 ng Angiotensin I ausgelösten Pressorresponses an der Ratte gemessen.

a) i.v. Applikation (30 Min. nach Applikation)

| Verbindung V | n | $X^1$ | Dosis | % Hemmung |
|---|---|---|---|---|
| $R^3$ | | | | |
| $C_2H_5$ ; | 1; | $=H_2$ | 100 µg/kg | 95% |
| $C_2H_5$ ; | 2; | $=H_2$ | 100 µg/kg | 95% |
| " ; | 2; | = O | 100 µg/kg | 95% |
| H ; | 3; | $=H_2$ | 100 µg/kg | 95% |

b) i.d. Applikaton (30 Min. nach Applikation)

| $R^3$ | n | $X^1$ | Dosis | % Hemmung |
|---|---|---|---|---|
| $C_2H_5$ | 1; | $=H_2$ | 1 mg/kg | 85—95% |
| $C_2H_5$ | 2; | $=H_2$ | 0.1 mg | 60—70% |

Die Verbindungen der Formel V mit $R^4$=Wasserstoff liegen als innere Salze vor. Falls beide Carboxylgruppen frei sind, können zusätzlich Alkali- und Calcium-, Magnesium- und Zinksalze und solche mit physiologisch unbedenklichen Aminen gebildet werden. Ferner kann die freie Aminogruppe mit einer Mineralsäure oder organischen Säure zu einem Salz umgesetzt werden.

Beispiel 1

1-(Di-(ethyloxy)ethyl)-cyclohexancarbonitril

Zu 312,5 ml (0,5 Mol) einer 15 %igen Lösung von n-Butyllithium in Hexan werden bei −10°C unter Schutzgas 51,7 ml (0,5 Mol) wasserfreies Diethylamin getropft. Man rührt 20 Min. und kühlt dann auf −70°C ab. Im Verlauf von 30 Min. werden 54,6 g Cyclohexancarbonitril eingetropft, nach weiteren 30 Min. innerhalb 1 Stunde 98,5 g Bromacetaldehyd-diethylacetal zugefügt und 25 Stunden bei tiefer Temperatur

belassen. Man erwärmt auf Raumtemperatur, gibt auf 100 g Eis, extrahiert zweimal mit 500 ml Essigester, trocknet die organische Phase über Natriumsulfat, engt im Vakuum ein und untewirft den Rückstand der Vakuumdestillation.

Ausbeute: 90 g (rund 80% d. Th.) Kp. 78—79°C bei 8 Torr.

Beispiel 2

1-Aminomethyl-1-(di(ethyloxy)ethyl)-cyclohexan

90 g Di-(ethyloxy)ethyl-cyclohexancarbonitril werden in 1 l Ethanol gelöst und mit 60 g Natrium versetzt. Nach Auflösung des Metalls werden 100 ml Wasser zugefügt und das Lösungsmittel weitgehend im Vakuum entfernt. Der Rückstand wird mit 300 ml Wasser versetzt und 3× mit 200 ml Ether extrahiert. Man trocknet die etherische Phase über Natriumsulfat, engt ein und destilliert im Vakuum.

Ausbeute 83 g (etwa 90% d.Th.) Kp. 69—72°C bei 8 Torr.

Beispiel 3

Spiro[4.5]-2-aza-decan-3-carbonitril

80,2 g Aminomethyl-di-(ethyloxy)ethyl-cyclohexan werden unter Schutzgas ($N_2$ oder Ar) in einem Gemisch aus 300 ml Ethanol und 300 ml 1n Salzsäure ca. 1 Stunde gerührt. Nach vollständiger Spaltung des Ausgangsproduktes wird auf 0°C gekühlt und die Lösung durch Zugabe von 2 n Natronlauge schnell auf pH 5 gebracht. Man versetzt sofort mit 300 ml Eisessig (pH etwa 3), kühlt auf −10°C und fügt 17,5 g Natriumcyanid zu. Das Reaktionsgefäß wird verschlossen und 5 Stunden bei Raumtemperatur belassen. Man kontrolliert mit Hilfe der Dünnschichtchromatographie (System Essigester/Petrolether 2:1) auf vollständige Umsetzung (Schiff'sche Base Rf=0,6—0,7; Aminonitril Rf=0,28) und engt die Reaktionslösung zur Trockene ein.

Das rohe Aminonitril wird alsbald nach Beispiel 4 oder 5 weiter verarbeitet.

Beispiel 4

Spiro[4.5]-2-aza-decan-3-carbonsäure

Die Hälfte des in Beispiel 3 erhaltenen Aminonitrils wird mit 250 ml 4n Salzsäure versetzt und 4 Stunden am Rückfluß erhitzt. Spuren von entweichender Blausäure werden auf geeignete Weise (Ausfrieren, Absorption in basischer Eisen(II)-salzlösung) unschädlich gemacht. Die Lösung wird neutralisiert zur Trockene gebracht und mehrfach mit n-Butanol extrahiert. Der Abdampfrückstand der organischen Phase wird.

a) zur Gewinnung des Hydrochlorids aus Chloroform-Diisopropyletherether kristallisiert und wenn nötig nochmals aus einem Gemisch mit Ethanol umgefällt oder

b) durch Ausrühren mit Ionemaustauscher, z.B. IR 45 (Amberlite[R]) in wäßriger Lösung gereinigt und das Zwitterion nach Entfernen des Wassers aus Ethanol/Ether kristallisiert.

Ausbeute nach a): 31—32 g (82%)

Schmp. 205°C (Zers.) Hydrochlorid

Beispiel 5

Spiro[4.5]-2-aza-decan-3-carbonsäurebenzylester-hydrochlorid

Die Hälfte des nach Beispiel 3 gewonnenen Aminosäurenitrils wird in 70 ml Benzylalkohol aufgenommen. Man leitet bei Raumtemperatur 5 Min. einen langsamen HCl-Gasstrom durch die Lösung, hält 2—3 Stunden bei Raumtemperatur, engt im Vakuum gut ein, versetzt mit wäßriger Bicarbonatlösung bis pH 8,5 erreicht ist und extrahiert den Benzylester in Essigester. Die organische Phase wird getrocknet, mit einer äquivalenten Menge etherischer Salzsäure versetzt und eingeengt. Der Rückstand kristallisiert aus Diisopropylether und kann aus Methylenchlorid/Diisopropylether umkristallisiert werden.

Ausbeute: 43 g (82%)

Schmp. 145°C (Zersetzung)

**Patentansprüche für die Vertragsstaaten: BE CH DE FR GB IT LI NL SE**

1. Verbindung der Formel I

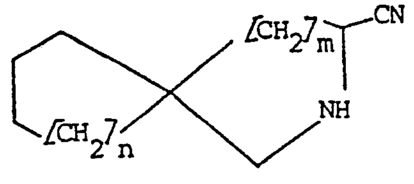

(I)

in welcher m eine ganze Zahl von 1 bis 3 und n eine ganze Zahl von 1 bis 4 bedeuten.

2. Verbindung der Formel I gemäß Anspruch 1, dadurch gekennzeichnet, daß m = 1 bedeutet.

3. Verfahren zur Herstellung einer Verbindung der Formel I gemäß Anspruch 1, dadurch

gekennzeichnet, daß man eine Verbindung der Formel II

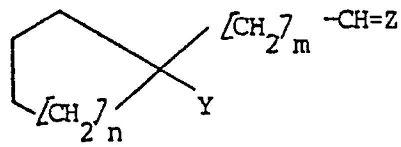

(II)

in welcher m und n die in Anspruch 1 genannten Bedeutungen haben, Y für Aminomethyl und Z für eine sauer abspaltbare, gegen metallorganische Reagentien inerte Oxo-Schutzgruppe stehen, aufeinanderfolgend mit a) Säuren und b) Cyanwasserstoff oder Cyaniden behandelt.

4. Verbindung der Formel II, in welcher m und n die in Anspruch 1 genannten Bedeutungen haben, Y Aminomethyl oder Cyan bedeutet und Z die in Anspruch 3 genannte Bedeutung hat, wobei 2-(1-aminomethylcyclopentyl)-acetaldehyd-diethyl-acetal, 2-(1-Cyanocyclopentyl)-acetaldehyd-diethyl-acetal, 2-(1-Cyanocyclohexyl)-acetaldehyd-1,3-dioxetan und 3-(1-Cyanocyclohexyl)-propionaldehyd-diethyl-acetal ausgenommen sind.

5. Verbindung der Formel II gemäß Anspruch 4, dadurch gekennzeichnet, daß m = 1 bedeutet.

6. Verbindung der Formel II gemäß Anspruch 5, dadurch gekennzeichnet, daß Y Aminomethyl bedeutet.

7. Verwendung einer Verbindung der Formel I gemäß Anspruch 1 zur Herstellung einer Verbindung der Formel III

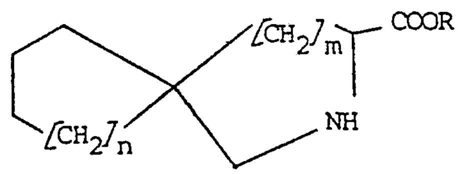

(III)

in welcher m und n die im Anspruch 1 genannten Bedeutungen haben und R für Wasserstoff, Alkyl mit 1 bis 10 C-Atomen, Aralkyl mit 7 bis 9 C-Atomen, Cycloalkyl mit 5 bis 10 C-Atomen, Alkylcycloalkyl mit 6 bis 12 C-Atomen oder Cycloalkylalkyl mit 6 bis 12 C-Atomen steht durch Solvolyse mit einer Verbindung der Formel ROH, in welcher R vorstehende Bedeutung hat.

8. Verwendung gemäß Anspruch 7, dadurch gekennzeichnet, daß R Wasserstoff bedeutet.

9. Verwendung gemäß Anspruch 7, dadurch gekennzeichnet, daß R die im Anspruch 7 genannten Bedeutungen mit Ausnahme der von Wasserstoff hat und daß die Solvolyse in Gegenwart wasserfreier Mineralsäuren durchgeführt wird.

**Patentansprüche für den Vertragsstaat: AT**

1. Verfahren zur Herstellung einer Verbindung der Formel I

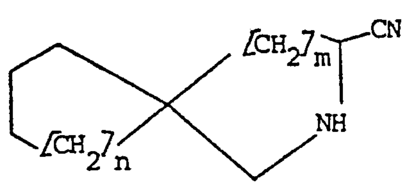

(I)

in welcher m eine ganze Zahl von 1 bis 3 und n eine ganze Zahl von 1 bis 4 bedeuten, dadurch gekennzeichnet, daß man eine Verbindung der Formel II

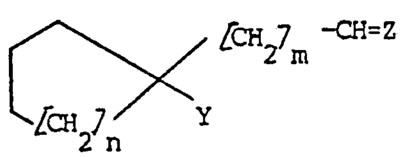

(II)

in welcher m und n die in Anspruch 1 genannten Bedeutungen haben, Y für Aminomethyl und Z für eine sauer abspaltbare, gegen metallorganische Reagentien inerte Oxo-Schutzgruppe stehen, aufeinderfolgend mit a) Säuren und b) Cyanwasserstoff oder Cyaniden behandelt.

2. Verfahren gemäß Anspruch 1, dadurch gekennzeichnet, daß m = 1 bedeutet.

3. Verfahren zur Herstellung einer Verbindung der Formel II, in welcher Z, m und n die in Anspruch 1

genannten Bedeutungen haben und Y Aminomethyl oder Cyan bedeutet, wobei die Verbindungen 2-(1-aminomethylcyclopentyl)-acetaldehyd-diethyl-acetal, 2-(1-Cyanocyclopentyl)-acetaldehyd-diethyl-acetal, 2-(1-Cyanocyclohexyl)-acetaldehyd-1,3-dioxetan und 3-(1-Cyanocyclohexyl)-propionaldehyd-diethyl-acetal ausgenommen sind, dadurch gekennzeichnet, daß dam ein Cycloalkancarbonitril der Formel IV,

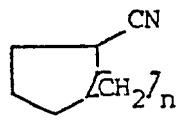

(IV)

in der n die vorstehend genannte Bedeutung hat, in α-Stellung metalliert, mit einem geschützten ω-Halogenaldehyd der Formel X—$[CH_2]_m$—CH = Z, in der m und Z vorstehende Bedeutung haben und X für Brom oder Chlor steht, unter Bildung einer Verbindung der Formel II, in der m, n und Z vorstehende Bedeutung haben und Y für Cyan steht, umsetzt und diese gegebenenfalls zu einer Aminomethylverbindung der Formel II reduziert.

4. Verfahren gemäß Anspruch 3, dadurch gekennzeichnet, daß m = 1 bedeutet.

5. Verfahren gemäß Anspruch 3, dadurch gekennzeichnet, daß Y Aminomethyl bedeutet.

6. Verwendung einer Verbindung der Formel I gemäß Anspruch 1 zur Herstellung einer Verbindung der Formel III

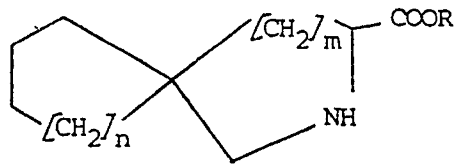

(III)

in welcher m und n die im Anspruch 1 genannten Bedeutungen haben und R für Wasserstoff, Alkyl mit 1 bis 10 C-Atomen, Aralkyl mit 7 bis 9 C-Atomen, Cycloalkyl mit 5 bis 10 C-Atomen, Alkylcycloalkyl mit 6 bis 12 C-Atomen oder Cycloalkylalkyl mit 6 bis 12 C-Atomen steht durch Solvolyse mit einer Verbindung der Formel ROH, in welcher R vorstehende Bedeutung hat.

7. Verwendung gemäß Anspruch 6, dadurch gekennzeichnet, daß R Wasserstoff bedeutet.

8. Verwendung gemäß Anspruch 6, dadurch gekennzeichnet, daß R die im Anspruch 6 genannten Bedeutungen mit Ausnahme der von Wasserstoff hat und daß die Solvolyse in Gegenwart wasserfreier Mineralsäuren durchgeführt wird.

**Revendications pour les Etats contractants: BE CH DE FR GB IT LI NL SE**

1. Composé de formule I

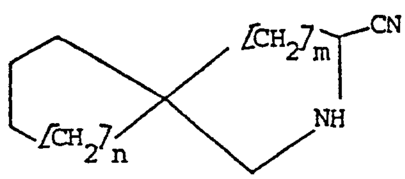

(I)

dans laquelle m représente un nombre entier valant de 1 à 3, et n représente un nombre entier valant de 1 à 4.

2. Composé de formule I selon la revendication 1, caractérisé en ce que m = 1.

3. Procédé pour la préparation d'un composé de formule I selon la revendication 1, caractérisé en ce que l'on traite successivement avec a) des acides et b) l'acide cyanhydrique ou des cyanures, un composé de formule II

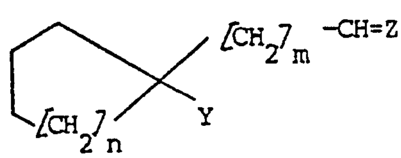

(II)

dans laquelle m et n ont les significations données dans la revendication 1, Y représente le groupe aminométhyle, et Z représente un groupe protecteur de fonction oxo, inerte vis-à-vis de réactifs organométalliques, séparable au moyen d'un acide.

4. Composé de formule II, dans lequel m et n ont les significtions données dans la revendication 1, Y représente le groupe aminométhyle ou cyano, et Z a la signification donée dans la revedication 3, à

l'exclusion du 2-(1-aminométhylcyclopentyl)-acétaldehyde-diéthylacétal, du 2-(1-cyanocyclopentyl)-acétaldéhyde-diéthylacétal, du 2-(1-cyanocyclohexyl)-acétaldéhyde-1,3-dioxétanne, et du 3-(1-cyanocyclohexyl)-propionaldéhyde-diéthylacétal.

5. Composé de formule II selon la revendication 4, caractérisé en ce que m = 1.

6. Composé de formule II selon la revendication 5, caractérisé en ce que Y représente le groupe aminométhyle.

7. Utilisation d'un composé de formule I selon la revendication 1, pour la préparation d'un composé de formule III

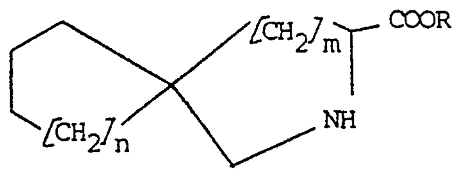

(III)

dans laquelle m et n ont les significations données dans la revendication 1, et R représente un atome d'hydrogène ou un groupe alkyle ayant de 1 à 10 atomes de carbone, aralkyle ayant de 7 à 9 atomes de carbone, cycloalkyle ayant de 5 à 10 atomes de carbone, alkylcycloalkyle ayant de 6 à 12 atomes de carbone ou cycloalkylalkyle ayant de 6 à 12 atomes de carbone, par solvolyse avec un composé de formule ROH, dans laquelle R a la signification donnée plus haut.

8. Utilisation selon la revendication 7, caractérisée en ce que R représentente un atome d'hydrogène.

9. Utilisation selon la revendication 7, caractérisée en ce que R a les significations données dans la revendication 7, à l'exception de celle d'un atome d'hydrogène, et en ce que l'on effectue la solvolyse en présence d'acides minéraux anhydres.

**Revendications pour l'Etat contractant: AT**

1. Procédé pour la préparation d'un composé de formule I

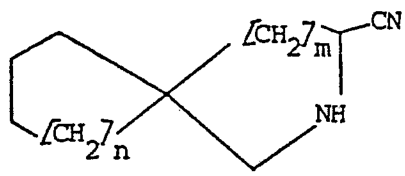

(I)

dans laquelle m représente un nombre entier valant de 1 à 3, et n représente un nombre entier valant de 1 à 4, caractérisé en ce que l'on traite successivement avec a) des acides et b) l'acide cyanhydrique ou des cyanures, un composé de formule II

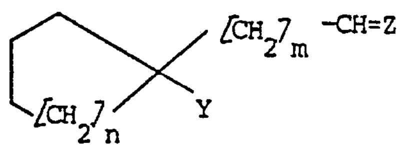

(II)

dans laquelle m et n ont significations données ci-dessus, Y représente le groupe aminométhyle, et Z représente un groupe protecteur de fonction oxo, inerte vis-à-vis de réactifs organométalliques, séparable au moyen d'un acide.

2. Procédé selon la revendication 1, caractérisé en ce que m = 1.

3. Procédé pour la préparation d'un composé de formule II dans lequel Z, m et n ont les significations données dans la revendication 1, et Y représente le groupe amino-méthyle ou cyano, à l'exclusion du 2-(1-aminométhylcyclopentyl)-acétaldehyde-diéthylacétal, du 2-(1-cyanocyclopentyl)-acétaldéhyde-diéthylacétal, du 2-(1-cyanocyclohexyl)-acétaldéhyde-1,3-dioxétanne, et du 3-(1-cyanocyclohexyl)-propionaldéhyde-diéthylacétal, caractérisé en ce que l'on soumet à une métallation en position α un cycloalcanecarbonitrile de formule IV

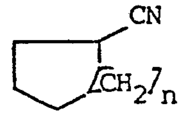

(IV)

dans laquelle n a la signification donnée plus haut, on le fait réagir avec un ω-halogéno-aldéhyde de formule $X—(CH_2)_m—CH=Z$, dans laquelle m et Z ont les significations précédentes et X représente le brome ou le chlore, avec formation d'un composé de formule II, dans lequel m, n et Z ont les significations

précédentes, et Y représente un groupe cyano, et éventuellement on le réduit pour aboutir à un composé aminométhyle de formule II.

4. Procédé selon la revendication 3, caractérisé en ce que m = 1.

5. Procédé selon la revendication 3, caractérisé en ce que Y représente le groupe aminométhyle.

6. Utilisation d'un composé de formule I selon la revendication 1, pour la préparation d'un composé de formule III

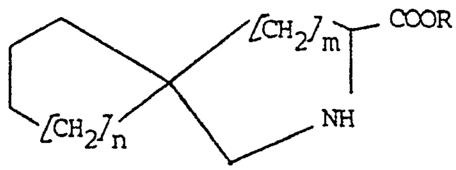

(III)

dans laquelle m et n ont les significations données dans la revendication 1, et R représente un atome d'hydrogène ou un groupe alkyle ayant de 1 à 10 atomes de carbone, aralkyle ayant de 7 à 9 atomes de carbone, cycloalkyle ayant de 5 à 10 atomes de carbone, alkylcycloalkyle ayant de 6 à 12 atomes de carbone ou cycloalkylalkyle ayant de 6 à 12 atomes de carbone, par solvolyse avec un composé de formule ROH, dans laquelle R a la signification précédente.

7. Utilisation selon la revendication 6, caractérisée en ce que R représente un atome d'hydrogène.

8. Utilisation selon la revendication 6, caractérisée en ce que R a les significations données dans la revendication 6, à l'exception de celle d'un atome d'hydrogène, et en ce que l'on effectue la solvolyse en présence d'acides minéraux anhydres.

**Claims for the Contracting States: BE CH DE FR GB IT LI NL SE**

1. A compound of the formula I

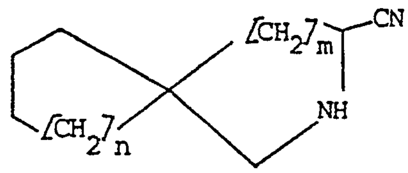

(I)

in which m is an integer from 1 to 3 and n is an integer from 1 to 4.

2. A compound of the formula I as claimed in claim 1, wherein m is 1.

3. A process for the preparation of a compound of the formula I as claimed in claim 1, which comprises treating a compound of the formula II

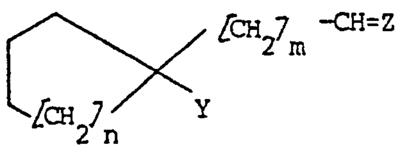

(II)

in which m and n are defined in claim 1, Y stands for aminomethyl and Z stands for an oxo-protecting group which can be split off by acids and is inert to organometallic reagants, in succession a) with acids and b) with hydrogen cyanide or cyanides.

4. A compound of the formula II, in which m and n are as defined in claim 1, Y is aminomethyl or cyano, and Z is as defined in claim 3, except 2-(1-aminomethylcyclopentyl)acetaldehyde diethyl acetal, 2-(1-cyanocyclopentyl)acetaldehyde diethyl acetal, 2-(1-cyanocyclohexyl)acetaldehyde 1,3-dioxetane and 3-(1-cyanocyclohexyl)propionaldehyde diethyl acetal.

5. A compound of the formula II as claimed in claim 4, wherein m is 1.

6. A compound of a formula II as claimed in claim 5, wherein Y is aminoethyl.

7. Use of a compound of the formula I as claimed in claim 1 for the preparation of a compound of the formula III

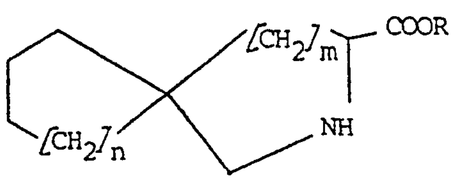

(III)

in which m and n are as defined in claim 1 and R stands for hydrogen, alkyl having from 1 to 10 carbon atoms, aralkyl having from 7 to 9 carbon atoms, cycloalkyl having from 5 to 10 carbon atoms,

alkylcycloalkyl or cycloalkylalkyl each having from 6 to 12 carbon atoms, by solvolysis of a compound of the formula ROH, in which R is as defined above.

8. Use as claimed in claim 7, wherein R is hydrogen.

9. Use as claimed in claim 7, wherein R is as defined in claim 7 with the exception of hydrogen, and the solvolysis is carried out in the presence of anhydrous mineral acids.

**Claims for the Contracting State: AT**

1. A process for the preparation of a compound of the formula I

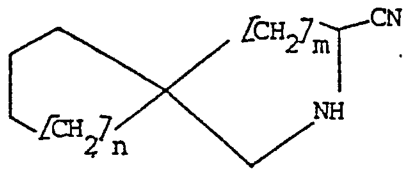

(I)

in which m is an integer from 1 to 3 and n is an integer from 1 to 4, which comprises treating a compound of the formula II

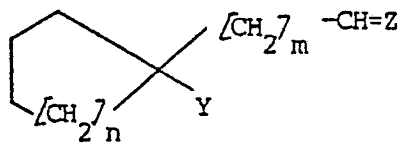

(II)

in which m and n are as defined in claim 1, Y stands for aminomethyl and Z stands for an oxo-protecting group which can be split off by acids and is inert to organometallic reagents, in succession a) with acids and b) with hydrogen cyanide or cyanides.

2. The process as claimed in claim 1, wherein m is 1.

3. A process for the preparation of a compound of the formula II, in which, m and n are as defined in claim 1, Y is aminomethyl or cyano except 2-(1-aminomethylcyclopentyl)acetaldehyde diethyl acetal, 2-(1-cyanocyclopentyl)acetaldehyde diethyl acetal, 2-(1-cyanocyclohexyl)acetaldehyde 1,3-dioxetane and 3-(1-cyanocyclohexyl)propionaldehyde diethyl acetal, which comprises metallating a cycloalkanecarbonitrile of the formula IV

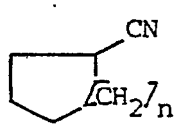

(IV)

in which n is as defined above, at the α-position, reacting the intermediate with a protected ω-haloaldehyde of the formula $X—[CH_2]_m—CH = Z$, in which m and z are as defined above and X stands for bromine or chlorine, to give a compound of the formula II, in which m, n and Z are as defined above and Y stands for cyano, and possibly reducing this compound to give an aminomethyl compound of the formula II.

4. The process as claimed in claim 3, wherein m is 1.

5. The process as claimed in claim 3, wherein Y is aminomethyl.

6. Use of a compound of the formula I as claimed in claim 1 for the preparation of a compound of the formula III

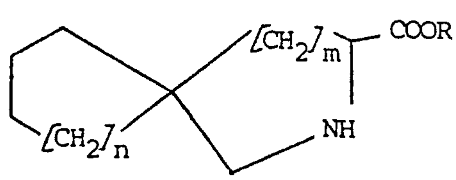

(III)

in which m and n are as defined in claim 1 and R stands for hydrogen, alkyl having from 1 to 10 carbon atoms, aralkyl having from 7 to 9 carbon atoms, cycloalkyl having from 5 to 10 carbon atoms, alkylcycloalkyl or cycloalkylalkyl each having from 6 to 12 carbon atoms, by solvolysis of a compound of the formula ROH, in which R is as defined above.

7. Use as claimed in claim 6, wherein R is hydrogen.

8. Use as claimed in claim 6, wherein R is as defined in claim 6 with the exception of hydrogen, and the solvolysis is carried out in the presence of anhydrous mineral acids.